# EUROPÄISCHE PATENTSCHRIFT

(11) **EP 3 886 738 B1**
(45) Veröffentlichungstag und Bekanntmachung des Hinweises auf die Patenterteilung: **12.08.2026**
(21) Anmeldenummer: 19809793.3
(22) Anmeldetag: 25.11.2019
(51) Int. Cl.: A61B 17/32, A61B 18/14, A61B 1/307

(54) **RESEKTOSKOP MIT ELEKTRODENINSTRUMENT IM AUSSENSCHAFT**
RESECTOSCOPE WITH ELECTRODE INSTRUMENT IN THE OUTER SHAFT
RÉSECTOSCOPE COMPRENANT UN INSTRUMENT DE TYPE ÉLECTRODE DANS LA TIGE EXTERNE

(30) Priorität: 27.11.2018 DE 102018129904
(43) Veröffentlichungstag der Anmeldung: 06.10.2021
(73) Patentinhaber: Olympus Winter & Ibe GmbH, 22045 Hamburg (DE)
(72) Erfinder: BROCKMANN, Christian, 21279 Hollenstedt (DE)
(74) Vertreter: Hoener, Matthias
(86) Internationale Anmeldenummer: PCT/EP2019/082476
(87) Internationale Veröffentlichungsnummer: WO 2020/109257

(56) Entgegenhaltungen:
- DE-A1- 102018 114 448
- US-A- 4 423 727
- US-A- 5 112 330
- US-A- 5 807 240
- US-A1- 2017 055 811
- US-A1- 2017 056 094

## Beschreibung

Die Erfindung betrifft ein Resektoskop der im Oberbegriff des Anspruches 1 genannten Art sowie ein elektrochirurgisches System der im Oberbegriff des Anspruches 9 genannten Art.

Resektoskope der gattungsgemäßen Art werden vor allem in der Urologie bei chirurgischen Arbeiten in der Blase und in der Urethra verwendet. Sie werden üblicherweise zur Resektion und Vaporisation von Gewebe, zum Beispiel von Gewebe im unteren Harntrakt, verwendet. Dazu umfassen die Resektoskope ein längsverschiebbares elektrochirurgisches Durchgangsinstrument, das nach dem Einführen des Resektoskops mit seinem distalen Arbeitsende aus dem distalen Ende des Schaftrohres des Resektoskops hervorgeschoben werden kann. Das elektrochirurgische Durchgangsinstrument kann an seinem distalen Arbeitsende eine elektrochirurgische Elektrode, zum Beispiel in Form eines Loops (Schlinge) oder PlasmaButtons, umfassen. Solche Instrumente sind zum Beispiel die OES PRO Resektoskope (Olympus) oder andere Dauerspül-Resektoskope nach Iglesias.

Die Instrumente enthalten eine Optik mittels derer der Eingriffsort während der Operation überwacht werden kann. Um die Anatomie im Sichtfeld aufzuweiten, während des Eingriffs entstehende lokale Blutungen wegzuspülen und das Gewebe vor Hitzeschäden durch die hochfrequente elektrochirurgische Anwendung zu schützen, sind die Resektoskope mit einer Spüleinrichtung versehen, die das vor dem distalen Schaftende liegende Gewebe dauerhaft umspült. In üblichen Spüleinrichtungen wird durch einen Innenschaft kontinuierlich Spülflüssigkeit geleitet, die am distalen Ende des Resektoskops austritt. Der Rückfluss der Spülflüssigkeit geschieht meist durch einen Spalt zwischen Innen- und Außenschaft. Zur Flüssigkeitsaufnahme weist der Außenschaft dafür zahlreiche Spülbohrungen auf.

In den derzeitigen Resektoskopen verlaufen sowohl das elektrochirurgische Durchgangsinstrument als auch die stabförmige Optik durch das auch für die Zufuhr von Spülflüssigkeit verwendete Innenrohr. Es hat sich gezeigt, dass ein solches System nicht optimal für die Führung des Spülflusses ist, da unter anderem die Elektrode direkt im Zustromkanal liegt und dadurch Turbulenzen in der Spülflüssigkeit unmittelbar vor deren Austritt aus dem Schaft erzeugen kann. Solche Turbulenzen können die Sicht auf das Operationsgebiet stark behindern. Darüber hinaus können sich in dem üblicherweise für den Rückstrom genutzten Ringspalt zwischen Innenrohr und Hüllrohr (Außenrohr) aufgrund der Wandreibungseffekte nur geringe Rückströmungsgeschwindigkeiten ausbilden. Dokument US 4,423,727 A offenbart ein Resektoskop mit einem Schaft, der ein äußeres Hüllrohr und ein Spülrohr umfasst. Das Resektoskop weist ein Teleskop und ein Behandlungsinstrument auf, die zwischen dem Spülrohr und dem Hüllrohr angeordnet sind. Das Spülrohr ist im Querschnitt C-förmig bzw. sichelförmig ausgebildet, wobei die konkave Kontur nach unten zeigt und oberhalb des Teleskops verschachtelt ist. Am vorderen Ende des Spülrohres sind Löcher vorgesehen, die klein genug dimensioniert sind, um zu verhindern, dass Blutgerinnsel das Rohr verstopfen.

Es besteht daher ein Bedarf an Systemen, in denen unerwünschte Verwirbelungen im Spülflüssigkeitszustrom vermieden oder reduziert werden und in denen die Rückstromgeschwindigkeit variabel ist.

### Beschreibung

Gelöst werden diese Aufgaben durch ein Resektoskop mit den Merkmalen von Anspruch 1 und ein elektrochirurgisches System mit den Merkmalen von Anspruch 10. Insbesondere werden die Aufgaben durch Führung des Elektrodeninstruments und der stabförmigen Optik parallel zu - d.h. außerhalb von - dem für die Spülflüssigkeitszufuhr genutzten Innenrohr (Spülrohr) gelöst. Die beiden Durchgangsinstrumente (Elektrodeninstrument und Optik) werden somit nicht mehr innerhalb des Innenrohrs geführt. Auf diese Weise wird das Entstehen von Verwirbelungen vermieden. Darüber hinaus kann der gesamte verbleibende Innenraum des Außenrohrs (Hüllrohrs) genutzt werden, um die verunreinigte Spülflüssigkeit aus dem Körper des Patienten herauszuführen.

In einem ersten Aspekt betrifft die Erfindung somit ein Resektoskop für die endoskopische Chirurgie mit einem rohrartigen Schaft, der ein langgestrecktes Hüllrohr und ein in dem Hüllrohr angeordnetes Spülrohr zur Zufuhr von Spülflüssigkeit sowie eine stabförmige Optik und ein Elektrodeninstrument umfasst, dadurch gekennzeichnet, dass die Optik und das Elektrodeninstrument zwischen der Außenwand des Spülrohrs und der Innenwand des Hüllrohres angeordnet sind. Eine Anordnung "zwischen" der Außenwand und der Innenwand bedeutet erfindungsgemäß, dass keine weiteren Trennelemente zwischen den genannten Elementen angeordnet sind. Insbesondere sind das Elektrodeninstrument und die Optik nur von dem Hüllrohr und nicht von einem weiteren Innenrohr umschlossen.

Das erfindungsgemäße Resektoskop ist für verschiedene Eingriffe in der endoskopischen Chirurgie geeignet, insbesondere in der elektrochirurgischen Chirurgie. So kann das Resektoskop beispielsweise für die Prostata-Resektion verwendet werden, da hier besonders starke Blutungen auftreten können. Gleichzeitig kann das Resektoskop aber auch für eine Vielzahl anderer Operationen eingesetzt werden, zum Beispiel für Blasen-Resektionen.

In üblicher Ausbildungsweise weist das Resektoskop einen rohrartigen Schaft auf. Neben diesem Schaftteil umfasst das Resektoskop zum Halten und Bedienen ein Griffsystem, das üblicherweise aus zwei Griffteilen besteht.

Der Endoskopschaft umfasst ein langgestrecktes Hüllrohr. Im Inneren des Hüllrohres ist ein Spülrohr (Innenrohr), eine stabförmige Optik und ein Elektrodeninstrument (elektrochirurgisches Durchgangsinstrument) angeordnet. Optik und Elektrodeninstrument sind radial neben dem Spülrohr im Inneren des Hüllrohres angeordnet. Mit anderen Worten durchlaufen Spülrohr, Optik und Elektrodeninstrument nebeneinander das Hüllrohr. Dabei ist insbesondere daran gedacht, dass das Spülrohr unterhalb einer Transversalebene des Resektoskops angeordnet ist. In einer räumlichen Orientierung kann ein Resektoskop durch eine das Schaftrohr in Längsrichtung und horizontal schneidende Transversalebene und eine senkrecht auf der Transversalebene stehende Sagittalebene in verschiedene Bereiche unterteilt sein, wobei die Längsachse eines Schaftrohres sowohl in der Sagittalebene als auch in der Transversalebene liegt. Die Transversalebene schneidet das Schaftrohr quer und in einer Gebrauchsstellung des Resektoskops in horizontaler Ausrichtung und die Sagittalebene schneidet das Schaftrohr senkrecht und in einer Gebrauchsstellung des Resektoskops in vertikaler Ausrichtung. Die Sagittalebene kann insbesondere parallel zu einer Bewegungsebene liegen, die bei einer Relativbewegung von, zum Beispiel für die Betätigung eines Schlittens des Resektoskops, schwenkbar zueinander an dem Resektoskop gelagerten Griffteilen beschrieben wird. In einer besonders bevorzugten Ausführungsform ist das Spülrohr in einer 6-Uhr-Position unterhalb der Optik angeordnet, sodass die Längsachsen des Spülrohrs und der Optik in der Sagittalebene des Resektoskops liegen.

Erfindungsgemäß sind die Optik und das Elektrodeninstrument infolge dieser Anordnung in dem Raum zwischen der Außenwand des Spülrohrs und der Innenwand des Hüllrohres angeordnet. Dieser Raum wird durch die Optik und das Elektrodeninstrument allerdings nicht vollständig ausgefüllt, sodass er auch für die Rückführung von Spülflüssigkeit in proximaler Richtung dienen kann. Der für den Rückfluss vorgesehene Teil des Raumes kann bewusst größer als üblich sein, um die Rückflussgeschwindigkeit zu begrenzen und den Geschwindigkeitsunterschied zwischen Zu- und Abfluss zu vergrößern. Im proximalen Bereich des Resektoskops kann der Volumenstrom des Abflusses darüber hinaus, zum Beispiel durch eine Verengung, verringert bzw. gebremst werden. Erfindungsgemäß ist die Zuflussgeschwindigkeit bevorzugt größer als die Abflussgeschwindigkeit. Auf diese Weise wird unter anderem ein unmittelbarer Rückfluss der Spülflüssigkeit verhindert.

Zur Zufuhr von Spülflüssigkeit dient erfindungsgemäß das in dem Hüllrohr angeordnete Spülrohr (Innenrohr). Durch das Einführen einer Spülflüssigkeit durch das Spülrohr in das Körperinnere während eines medizinischen Eingriffs wird sichergestellt, dass das medizinische Fachpersonal während der Behandlung über die Optik eine freie Sicht auf den zu behandelnden Bereich hat. Durch diese Spülflüssigkeit können beispielsweise Gewebestücke, die während der Resektoskopie freigesetzt werden, weggespült werden. Des Weiteren dient die Spülflüssigkeit dazu, Trübungen, die beispielsweise durch Blut verursacht werden, aus dem Blickfeld der Optik zu entfernen. Die Spülflüssigkeit wird über das Spülrohr dem Körperinneren zugeführt, während der Abfluss der verunreinigten Spülflüssigkeit vorzugsweise durch den Raum zwischen der Innenwand des Hüllrohres und der Außenwand des Spülrohres erfolgt. Das Spülrohr ist daher erfindungsgemäß so ausgebildet, dass es von Spül- und/oder Körperflüssigkeiten durchflossen werden kann, vorzugsweise in distaler Richtung.

Für gute Sichtbedingungen während der Operation ist es wesentlich, dass die Spülflüssigkeit beim Eintritt in den Körperhohlraum eine laminare Strömung ausbildet, die wenigstens nahezu parallel zu einer Längsachse des Schaftes bzw. einer optischen Achse der Optik verläuft und eine visuelle Kontrolle der ausgefahrenen Elektrode zulässt. Sobald die Spülflüssigkeit nicht laminar oder sogar turbulent strömt, kann die Sicht durch die Optik derart schlecht werden, dass eine Operation nicht durchführbar ist. Erfindungsgemäß ist es zu diesem Zweck vorgesehen, dass das Spülrohr in seinem distalen Endbereich eine Düse aufweist, mittels derer ein in distale Richtung fließender Flüssigkeitsstrom gelenkt und/oder beschleunigt werden kann. Die Düse kann durch eine Verengung des Spülrohres in dessen distalen Endbereich gebildet sein oder durch ein separates Düsenteil, das am distalen Ende des Spülrohres angeordnet ist. Die Düse beschleunigt das Spülmedium und kann es in die Mitte des Sichtfeldes lenken.

Die Düse am distalen Ende des Spülrohres erzielt eine von Turbulenzen freie Strömung.

Um das Spülrohr neben der Optik platzsparend in das Hüllrohr einzupassen, hat das Spülrohr einen Querschnitt mit einem konvex gewölbten Abschnitt und einem konkav gewölbten Abschnitt, d.h. einen Querschnitt in Sichelform. Die Sichelform weist vorzugsweise gerundete Ecken bzw. Spitzen auf. Der konkav gewölbte Abschnitt - das Innere der Sichelform - grenzt dabei bevorzugt mindestens abschnittsweise an die Optik an. Der konvex gewölbte Abschnitt grenzt bevorzugt an die Innenwand des Hüllrohres an.

Um die Gefahr von Verwirbelungen im Zustrom der Spülflüssigkeit weiter zu reduzieren, weist das Spülrohr auf einem möglichst langen Abschnitt vor seinem distalen Ende bzw. vor der Düse in seinem distalen Endbereich, sofern das Spülrohr eine solche / einen solchen umfasst, einen in Form und Größe gleichbleibenden Querschnitt auf. So ist es beispielsweise bevorzugt, dass das Spülrohr auf mindestens 60%, vorzugsweise mindestens 70%, stärker bevorzugt mindestens 80%, der Länge des Resektoskopschafts einen in Größe und Form gleichbleibenden Querschnitt aufweist. Vorzugsweise ist der Querschnitt insbesondere in den distalen 60%, bzw. 70%, bzw. 80%, des Schaftes, mit Ausnahme einer vorhandenen Düse, gleichbleibend.

Die Verwirbelungsfreiheit wird erfindungsgemäß ferner dadurch sichergestellt, dass innerhalb des Spülrohrs keine Durchgangsinstrumente, insbesondere kein Elektrodeninstrument und keine Optik, angeordnet sind. In üblichen Resektoskopen führt die Anordnung dieser Instrumente im Spülstrom regelmäßig zu störenden Turbulenzen im Spülstrom.

Wie an anderer Stelle beschrieben, sind neben dem Spülrohr im Hüllrohr eine Optik und ein Elektrodeninstrument angeordnet. Geeignete Elektrodeninstrumente zur Anordnung sind Fachleuten bekannt. Das Elektrodeninstrument weist in der Regel einen langgestreckten Instrumentenschaft auf und mindestens eine am distalen Endbereich angeordnete Elektrode. Die Elektrode kann beispielsweise als Plasmabutton, Schneidschlinge oder ein anderes elektrochirurgisches Schneidwerkzeug ausgebildet sein, wobei eine Schneidschlinge bevorzugt ist. Das Elektrodeninstrument ist vorzugsweise ein bipolares Instrument. Es ist allerdings auch denkbar, in dem erfindungsgemäßen Resektoskop ein monopolares Elektrodeninstrument zu verwenden.

Das Elektrodeninstrument kann zwei Gabelrohre aufweisen. Ein derartiger Aufbau ist für viele Elektrodeninstrumente, insbesondere bipolare Instrumente mit zum Beispiel einer Schneidschlinge am distalen Ende, bekannt. Üblicherweise verlaufen diese Gabelrohre in dem proximalen und mittleren Schaftbereich des Elektrodeninstruments relativ dicht nebeneinander und laufen erst im distalen Endbereich des Elektrodeninstruments auseinander, sodass die distalen Enden der Gabelrohre eine Elektrode, beispielsweise in Form einer Schlingenelektrode oder eines Plasmabuttons, zwischen sich aufnehmen können. Um erfindungsgemäß die Anordnung des Elektrodeninstruments in dem Raum zwischen dem Hüllrohr und dem Spülrohr zu erleichtern, kann es vorgesehen sein, dass die Gabelrohre des Elektrodeninstruments erst im proximalen Endbereich des Elektrodeninstruments oder gar nicht zusammenlaufen. Auf diese Weise wird auch der für das Spülrohr zur Verfügung stehende Raum maximiert. Der Abstand zwischen den Gabelrohren ist daher erfindungsgemäß vom distalen Ende des Elektrodeninstruments aus bevorzugt auf mindestens 60%, vorzugsweise mindestens 70%, stärker bevorzugt mindestens 80 % des Schaftes des Elektrodeninstruments konstant. Die Gabelrohre des Elektrodeninstruments verlaufen daher beispielsweise bis zu dem Transporteur des Resektoskops gerade (und zueinander parallel), sodass der für das Spülrohr verbleibende Raum maximiert wird und Störstellen für den Flüssigkeitsausstrom vermieden werden. Diese Ausbildung der Gabelrohre hat den zusätzlichen Vorteil, dass Kosten in der Montage gespart werden.

Die Gabelrohre des Elektrodeninstruments, z. B. eines Elektrodeninstruments mit zwei Gabelrohren, verlaufen vorzugsweise entlang der Innenwand des Hüllrohres, wobei es besonders bevorzugt ist, dass die Optik zwischen den beiden Gabelrohren eines Elektrodeninstruments angeordnet ist. Auf diese Weise wird im Innenraum des Hüllrohres besonders viel Platz für das Spülrohr geschaffen. Die beiden Gabelrohre des beispielhaften Elektrodeninstruments können beispielsweise zwischen der 9- und der 10-Uhr-Position und zwischen der 2- und der 3-Uhr-Position angeordnet sein.

Das erfindungsgemäß verwendete Elektrodeninstrument kann ein oder mehrere Halteelemente zur radialen Abstützung aufweisen. Auf diese Weise wird ein unerwünschtes seitliches Verschieben der Elektrode während einer Operation verhindert. Gleichzeitig ist das Elektrodeninstrument in axialer Richtung verschiebbar. So kann die Elektrode, die am distalen Ende des Elektrodeninstruments angeordnet ist, beispielsweise zum Abtragen von Gewebe verwendet werden. Die axiale Verschiebbarkeit des Elektrodeninstruments wird durch die Haltelemente nicht beeinträchtigt.

Das oder die Halteelemente können das Elektrodeninstrument radial abstützend mit einem der anderen Elemente des Schafts verbinden, beispielsweise mit der Optik, dem Spülrohr oder dem Hüllrohr. "Verbinden" bedeutet in diesem Falle, dass die Haltelemente radial an das jeweilige Element angrenzen, aber in axialer Richtung weiterhin gegenüber dem Element verschiebbar sind. Das oder die Halteelemente können beispielsweise an die Außenwand der stabförmigen Optik angrenzen. In einer alternativen und bevorzugten Ausführungsform grenzen das oder die Halteelemente an die Innenwand des Hüllrohres an. Hierzu weisen die Halteelemente bevorzugt einen teilkreisförmigen Querschnitt auf. Durch den teilkreisförmigen Querschnitt ist das oder die Halteelemente formkomplementär zu dem angrenzenden Element. So können das oder die Haltelemente, vorzugsweise zwei Halteelemente, beispielsweise die Optik auf einem Abschnitt ihres Schaftteils teilkreisförmig umschließen. Alternativ können das oder die Halteelemente, vorzugsweise ein Halteelement, auf einem Abschnitt des Hüllrohres an der Innenwand des Hüllrohres entlang teilkreisförmig verlaufen. In diesem Falle kann beispielsweise ein Halteelement derart ausgebildet sein, dass es entlang der Innenwand des Hüllrohres von einem Gabelrohr zum anderen Gabelrohr verläuft und die Gabelrohre miteinander stabilisierend verbindet und gleichzeitig radial gegen die Innenwand abstützt. Das Halteelement kann zwischen dem jeweiligen Gabelrohr und der Innenwand des Hüllrohres angeordnet sein.

Das Hüllrohr kann an seinem distalen Endbereich eine Isolierspitze aufweisen. Diese verhindert Kurzschlüsse zwischen der Elektrode und proximaler angeordneten, elektrisch leitfähigen Elementen des Resektoskops, wie beispielsweise des Spülrohrs. Dem Fachmann sind geeignete Materialien zur Ausbildung solcher Isolierspitzen bekannt. So kann die Isolierspitze beispielsweise aus Keramik, Kunststoff oder Glas sein. Während es üblich und auch im Rahmen der Erfindung denkbar ist, die Isolierspitze mit einer Schnabelform auszubilden, ist es erfindungsgemäß bevorzugt, dass die Isolierspitze in distale Richtung entlang ihres gesamten Umfangs eine gleichbleibende Länge aufweist.

Die in dem Schaft angeordnete Optik des vorliegenden Resektoskops ist eine stabförmige Optik, die ihrerseits einen Schaftbereich aufweist, der den Resektoskopschaft durchläuft. Mittels der Optik wird dem Nutzer ermöglicht, den Eingriffsort und den vorgenommenen elektrochirurgischen Eingriff visuell zu überwachen. Die Optik kann ein Linsen-basiertes Optiksystem oder eine Faseroptik umfassen. In ihrem proximalen Endbereich umfasst die Optik ein Okular oder eine Verbindung zu einem Kamerakopf. An ihrem distalen Ende ist die Optik in der Regel durch ein Schutzglas geschützt, das gleichzeitig als Filter wirken kann. Die Optik kann eine schräg blickende Optik sein, um einen besonders guten Blick auf den Eingriffsort zu gewährleisten.

In einem zweiten Aspekt betrifft die Erfindung ein elektrochirurgisches System, das ein erfindungsgemäßes Resektoskop und eine Spülflüssigkeitszufuhreinrichtung umfasst, die mit dem Spülrohr des Resektoskops verbunden ist. Die Verbindung zu dem Spülrohr stellt sicher, dass Spülflüssigkeit aus der Spülflüssigkeitszufuhreinrichtung in distale Richtung durch das Spülrohr fließen kann. Dem proximalen Ende des Resektoskops bzw. des Schaftes, insbesondere des Spülrohrs, ist somit eine Spülflüssigkeitszufuhreinrichtung zuordbar. Die Spülflüssigkeitszufuhreinrichtung ist in der Lage, die Spülflüssigkeit mit einem vorbestimmbaren Druck in das Körperinnere zu leiten. Die Spülflüssigkeitszufuhreinrichtung kann zu diesem Zweck beispielsweise ausschließlich mit hydrostatischem Druck arbeiten, d.h. mit einem Flüssigkeitsreservoir ausgestattet sein, das oberhalb des Resektoskops angeordnet ist. Alternativ oder zusätzlich kann die Spülflüssigkeitszufuhreinrichtung eine Pumpe umfassen, mittels derer die Spülflüssigkeit durch das Spülrohr in das Körperinnere gepumpt werden kann.

In der Regel wird der Abfluss überschüssiger Flüssigkeit durch den Raum zwischen Spülrohr und Hüllrohr spontan erfolgen. Es ist aber auch möglich, den Abfluss durch Anlegung eines leichten Unterdrucks sicherzustellen. Zu diesem Zweck kann das elektrochirurgische System ferner eine Spülflüssigkeitsabflusseinrichtung umfassen.

### Kurze Beschreibung der Figuren

In den Zeichnungen sind Ausführungsbeispiele der Erfindung schematisch dargestellt. Es zeigen:
- **Fig. 1**: eine schematische, seitliche Schnittdarstellung eines erfindungsgemäßen Resektoskops;
- **Fig. 2**: eine Frontalansicht auf den Resektoskopschaft aus distaler Richtung, wobei ausschließlich Schaftelemente des Resektoskops dargestellt sind;
- **Fig. 3**: eine schematische, seitliche Schnittdarstellung eines erfindungsgemäßen Resektoskops, dessen Spülrohr an seinem distalen Ende eine Düse aufweist;
- **Fig. 4**: eine schematische, seitliche Schnittdarstellung einer alternativen Ausführungsform eines erfindungsgemäßen Resektoskops, dessen Spülrohr an seinem distalen Ende einen Diffusor aufweist; und
- **Fig. 5**: eine schematische, seitliche Schnittdarstellung eines erfindungsgemäßen elektrochirurgischen Systems, das eine Spülflüssigkeitszufuhreinrichtung aufweist.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

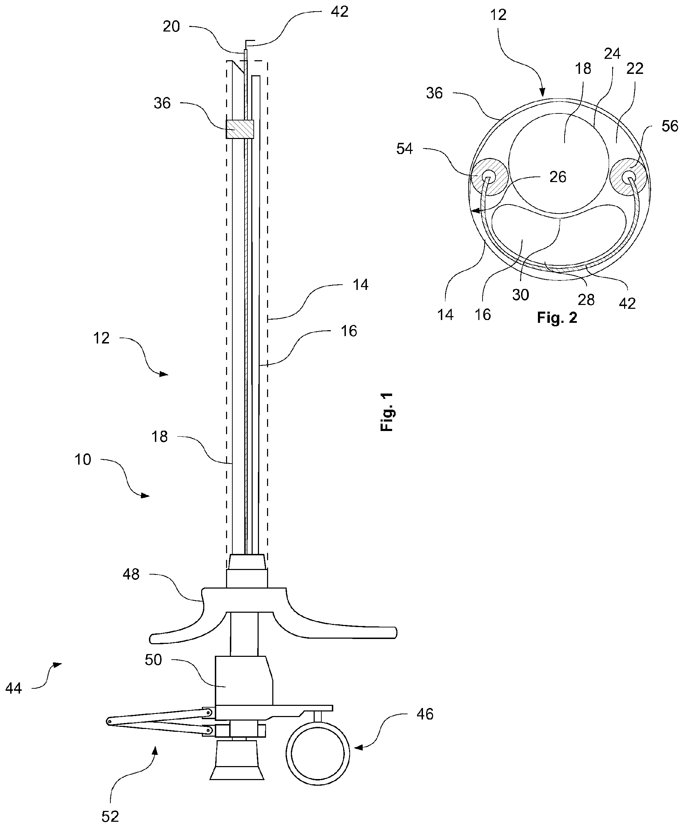

Fig. 1 zeigt eine Schnittdarstellung eines erfindungsgemäßen Resektoskops 10. Fig. 2 zeigt eine Frontalansicht auf den Resektoskopschaft 12 aus distaler Richtung, wobei ausschließlich Schaftelemente des Resektoskops 10 aus Fig. 1 dargestellt sind und keine Elemente des Handgriffs 44. In üblicher Ausbildungsweise umfasst das Resektoskop 10 einen Handgriff 44 und einen Schaft 12. Der Handgriff 44 ist zum Halten des Resektoskops 10 in einer Hand und zur, vorzugsweise einhändigen, Betätigung der durch den Schaft 12 hindurch verlaufenden Durchgangsinstrumente ausgebildet.

Das dargestellte Resektoskop 10 weist einen passiven Transporteur auf, bei dem der Schlitten 50 durch Relativbewegung der proximal von dem Schaft 12 angeordneten Griffteile 46 und 48 zueinander gegen eine von einer Federbrücke 52 aufgebrachten Federkraft in distale Richtung gegen das distale, erste Griffteil 48 verschoben wird. Bei der Verschiebung des Schlittens 50 in distale Richtung gegen das Griffteil 48 wird das Elektrodeninstrument 20 in nicht dargestellter Weise zwangsgeführt nach distal verschoben. Bei einer Entlastung der Handgriffteile 46, 48 zwingt die von der Federbrücke 52 erzeugte Federkraft den Schlitten 50 zurück in seine Ruheposition, wobei das Elektrodeninstrument 20 in proximale Richtung gezogen wird. Bei der Rückverschiebung des Schlittens 50 kann ohne Handkraft des Operateurs, also passiv, ein elektrochirurgischer Eingriff mit dem Elektrodeninstrument 20 vorgenommen werden.

Der Schaft 12 des Resektoskops 10 umfasst ein Hüllrohr 14, in dessen Inneren mehrere längliche Durchgangsinstrumente verlaufen, insbesondere die langgestreckte Optik 18, das Elektrodeninstrument 20 und das Spülrohr 16. Es ist in Fig. 1 und 2 erkennbar, dass im Inneren des Spülrohrs 16 keine weiteren Durchgangsinstrumente verlaufen. So sind insbesondere das Elektrodeninstrument 20 und die Optik 18 neben dem Spülrohr 16 in dem Hüllrohr 14 angeordnet. Das Elektrodeninstrument 20 ist dabei zur Stabilisierung durch ein Haltelement 36 gegen eine radiale Verschiebung gesichert. Das Halteelement 36 weist einen teilkreisförmigen Querschnitt auf, der in dem vorliegenden Instrument an der Innenwand 26 des Hüllrohres 14 etwa entlang des halben Innenumfangs der Innenwand 26 anliegt. Der Querschnitt des Halteelements 36 ist im vorliegenden Fall somit in etwa halbkreisförmig, wie in Fig. 2 erkennbar. Mit anderen Worten ist das Halteelement 36 teilweise formkomplementär zu der Innenwand 26 des Hüllrohres 14. Dadurch ist das Halteelement 36 in axiale Richtung im Inneren des Hüllrohres 14 verschiebbar, während es radial abgestützt wird.

Das Halteelement 36 verbindet entlang des Innenumfangs des Hüllrohres 14 die beiden Gabelrohre 54, 56 des Elektrodeninstruments miteinander. Die Verbindung zwischen dem Halteelement 36 und den beiden Gabelrohren 54, 56 kann auf übliche Weise, wie beispielsweise Laserstahlschweißen hergestellt werden. Alternativ können Gabelrohre 54, 56 und das Halteelement 36 auch aus einem Stück hergestellt werden.

An seinem distalen Ende weist das Elektrodeninstrument 20 eine Elektrode auf, die im vorliegenden Fall als Schlingenelektrode 42 bzw. Schneidschlinge ausgebildet ist. Das Instrument ist als bipolares Instrument ausgebildet und mit einer nicht dargestellten Gegenelektrode versehen. Mittels der Schlingenelektrode 42 ist das medizinische Fachpersonal in der Lage, bei einem chirurgischen Eingriff Gewebe von der Eingriffsstelle abzutragen.

Das Spülrohr 16 weist einen Querschnitt mit einem konvex gewölbten Abschnitt 28 und einem konkav gewölbten Abschnitt 30 auf, d.h. einen Querschnitt in Sichelform mit gerundeten Spitzen. Der konkav gewölbte Abschnitt 30 - das Innere der Sichelform - grenzt über die Länge des Schaftes 12 an den Schaft der Optik 18 an. Der konvex gewölbte Abschnitt 28 grenzt an die Innenwand 26 des Hüllrohres 14 an. Auf diese Weise wird der zur Verfügung stehende Innenraum des Hüllrohres 14 möglichst platzsparend genutzt und das Innenvolumen des Spülrohres 16 maximiert. Durch das Spülrohr 16 kann in distaler Richtung Spülflüssigkeit an die Eingriffsstelle geleitet werden. Der Rückfluss der verunreinigten Spülflüssigkeit erfolgt durch den leeren Raum 22 der im Inneren des Hüllrohres 14 neben den dort angeordneten Durchgangsinstrumenten verbleibt, d.h. dem Raum 22 der neben Optik 18, Spülrohr 16 und Elektrodeninstrument 20 verbleibt.

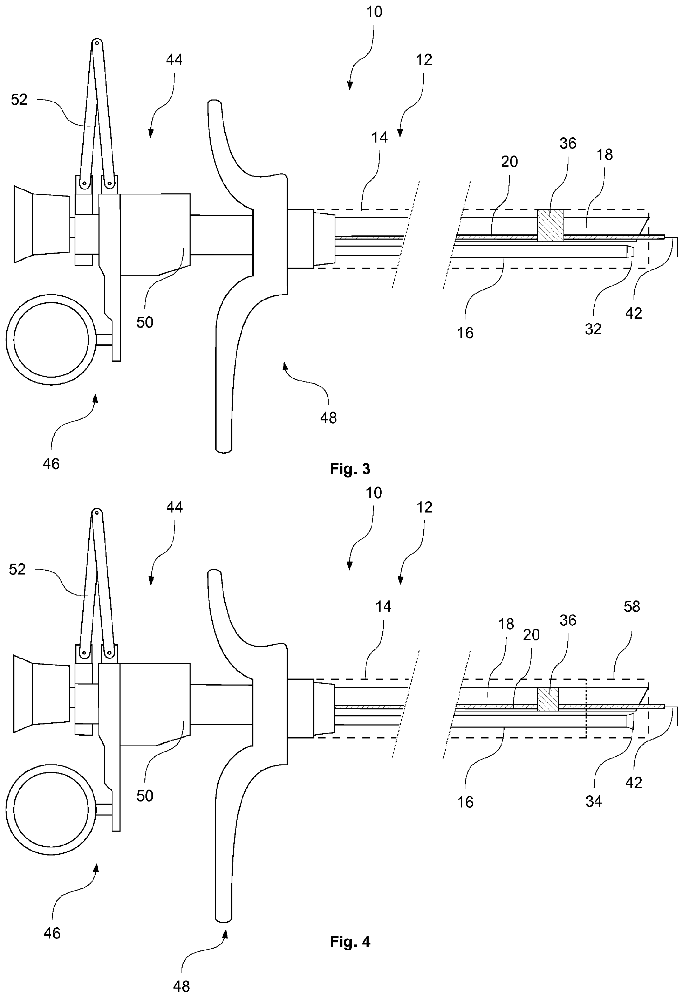

Die Fig. 3 und 4 zeigen zwei unterschiedliche Ausführungsvarianten des in den Fig. 1 und 2 gezeigten Resektoskops. Das in Fig. 3 dargestellte Resektoskop 10 weist neben den vorstehend beschriebenen Elementen eine Düse 32 an dem distalen Ende des Spülrohres 16 auf. Mittels der Düse 32 kann der in distale Richtung fließende Flüssigkeitsstrom an die Eingriffsstelle gelenkt werden. In einigen Ausführungsformen kann die Richtung, in welche die Düse 32 den Flüssigkeitsstrom lenkt, manuell oder automatisch verstellbar sein. Auf diese Weise kann das medizinische Fachpersonal die Richtung des Flüssigkeitsstroms während der Operation einstellen und bei Bedarf ändern. Darüber hinaus wirkt die Düse 32 beschleunigend auf den Flüssigkeitsstrom, sodass ein unmittelbarer Rückfluss der Spülflüssigkeit verhindert wird.

In der alternativen, in Fig. 4 gezeigten Ausführungsform, weist das Spülrohr 16 an seinem distalen Ende einen Diffusor 34 auf. Mittels des Diffusors 34 kann der in distale Richtung fließende Flüssigkeitsstrom gelenkt und die Geschwindigkeit des Flüssigkeitsstroms reduziert werden. Ein Diffusor 34 kann beispielsweise bei Eingriffen, die nur geringe Blutungen zur Folge haben, nützlich sein. Durch den entschleunigten Flüssigkeitsstrom ist der Eingriff mit dieser Ausführungsform besonders schonend.

Darüber hinaus weist das Resektoskop 10 in Fig. 4 ein elektrisch isolierendes Element an der Spitze des Hüllrohres 14 eine Isolierspitze 58 auf, die als Keramikspitze ausgebildet ist. Ein Kurzschluss zwischen der Schlingenelektrode 42 und dem distalen Ende des Spülrohres 16 und anderen Elementen wird auf diese Weise verhindert.

Schließlich weist das in Fig. 4 dargestellte Resektoskop 10 ein Elektrodeninstrument 20 mit Halteelementen 36 auf, die sich von dem in Fig. 1 bis 3 dargestellten unterscheiden. Das hier gezeigte Elektrodeninstrument 20 weist zwei Halteelemente 36 auf, von denen nur eines in der Fig. 4 erkennbar ist. Die Halteelemente 36 stützen das Elektrodeninstrument 20 nicht an der Innenwand 26 des Hüllrohres 14 ab, sondern an der Außenwand 24 der Optik 18. Der Querschnitt der beiden Halteelemente 36 weist ebenfalls einen teilkreisförmigen Querschnitt auf, der aber kleiner ist als der in Fig. 2 gezeigte, etwa halbkreisförmige Querschnitt. Die Halteelemente 36 grenzen formkomplementär an die Außenwand 24 der Optik 18 an und sind gegenüber der Optik 18, gemeinsam mit dem restlichen Elektrodeninstrument 20 parallel, d.h. in axiale Richtung, verschiebbar.

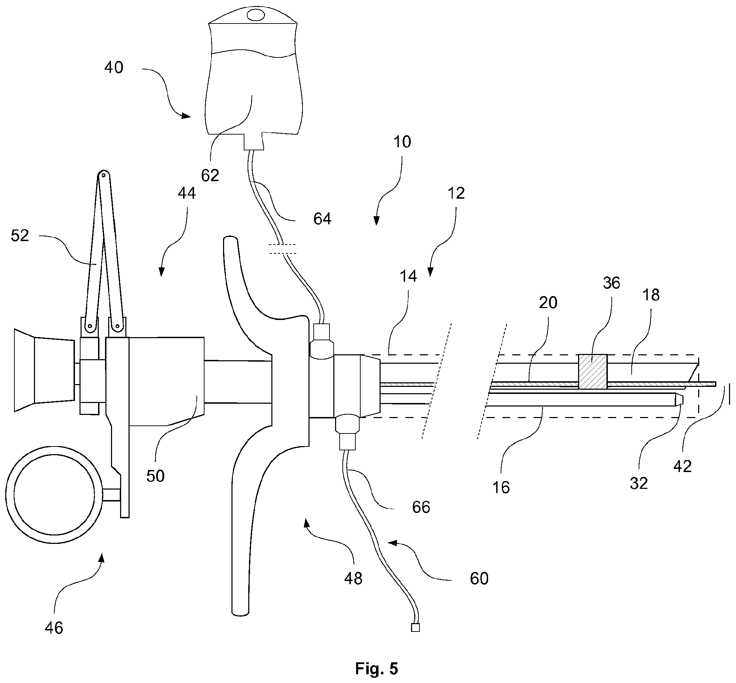

Fig. 5 zeigt ein eine schematische, seitliche Schnittdarstellung eines erfindungsgemäßen elektrochirurgischen Systems, das eine Spülflüssigkeitszufuhreinrichtung 40 aufweist. In der dargestellten Ausführungsform umfasst die Spülflüssigkeitszufuhreinrichtung 40 ein Flüssigkeitsreservoir 62 und einen Schlauch 64 durch den die Spülflüssigkeit in das Spülrohr 16 des Resektoskops 10 strömen kann. In der gezeigten Ausführungsform fließt die Spülflüssigkeit durch den hygrostatischen Druck der Flüssigkeit in dem Flüssigkeitsreservoir 62 in das Spülrohr. Durch Veränderung der Höhe des Flüssigkeitsreservoirs 62 in Relation zu dem Resektoskop 10 kann die Geschwindigkeit der Spülflüssigkeit angepasst werden. Nach dem Erreichen der Eingriffsstelle kann die Spülflüssigkeit durch den Raum 22 zwischen der Innenwand 26 des Hüllrohres 14 und den darin angeordneten Durchgangsinstrumenten wieder abfließen. Die Spülflüssigkeitsabflusseinrichtung 60 umfasst daher im vorliegenden Falle einen Schlauch 66 durch den die Flüssigkeit abfließen kann.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird. Die vorliegende Offenbarung schließt sämtliche Kombinationen der vorgestellten Einzelmerkmale ein.

### Bezugszeichenliste

| | | | |
|---|---|---|---|
| 10 | Resektoskop | 48 | Griffteil |
| 12 | Schaft | 50 | Schlitten |
| 14 | Hüllrohr | 52 | Federbrücke |
| 16 | Spülrohr | 54 | Gabelrohr |
| 18 | Optik | 56 | Gabelrohr |
| 20 | Elektrodeninstrument | 58 | Isolierspitze |
| 22 | Raum | 60 | Spülflüssigkeitsabflusseinrichtung |
| 24 | Außenwand Optik | 62 | Flüssigkeitsreservoir |
| 26 | Innenwand Hüllrohr | 64 | Schlauch |
| 28 | konvex gewölbter Abschnitt | 66 | Schlauch |
| 30 | konkav gewölbter Abschnitt | | |
| 32 | Düse | | |
| 34 | Diffusor | | |
| 36 | Halteelement | | |
| 38 | elektrochirurgisches System | | |
| 40 | Spülflüssigkeitszufuhreinrichtung | | |
| 42 | Schlingenelektrode | | |
| 44 | Handgriff | | |
| 46 | Griffteil | | |

## Patentansprüche

1. Resektoskop (10) für die endoskopische Chirurgie mit einem rohrartigen Schaft (12), der ein langgestrecktes Hüllrohr (14) und ein in dem Hüllrohr (14) angeordnetes Spülrohr (16), das der Zufuhr von Spülflüssigkeit dient, sowie eine stabförmige Optik (18) und ein Elektrodeninstrument (20) umfasst, wobei die Optik (18) und das Elektrodeninstrument (20) zwischen der Außenwand (24) des Spülrohrs (16) und der Innenwand (26) des Hüllrohres (14) angeordnet sind, wobei die durch das Spülrohr (16) in einen Körper eines Patienten zugeführte Spülflüssigkeit durch das Hüllrohr (14) wieder aus dem Körper herausführbar ist und wobei das Spülrohr (16) einen Querschnitt mit einem konvex gewölbten Abschnitt (28) und einem konkav gewölbten Abschnitt (30) aufweist, wobei das Spülrohr (16) in seinem distalen Endbereich eine Düse (32) aufweist, mittels derer ein in distale Richtung fließender Flüssigkeitsstrom gelenkt und beschleunigt werden kann.

2. Resektoskop (10) nach Anspruch 1, wobei der konvex gewölbte Abschnitt (28) an die Innenwand (26) des Hüllrohres (14) angrenzt.

3. Resektoskop (10) nach einem der Ansprüche 1 bis 2, wobei das Spülrohr (16) in seinem distalen Endbereich einen Diffusor (34) aufweist.

4. Resektoskop (10) nach einem der vorangegangenen Ansprüche, wobei das Elektrodeninstrument (20) ein oder mehrere Halteelemente (36) zur radialen Abstützung aufweist.

5. Resektoskop (10) nach Anspruch 4, wobei das oder die Halteelemente (36) an die Innenwand (26) des Hüllrohres (14) angrenzen.

6. Resektoskop (10) nach Anspruch 4, wobei das oder die Halteelemente (36) an die Außenwand (24) der stabförmigen Optik (18) angrenzen.

7. Resektoskop (10) nach einem der vorangegangenen Ansprüche, wobei das Spülrohr (16) auf mindestens 60%, vorzugsweise mindestens 70%, der Länge des Schafts (12) einen in Größe und Form gleichbleibenden Querschnitt aufweist.

8. Resektoskop (10) nach einem der vorangegangenen Ansprüche, wobei innerhalb des Spülrohrs (16) keine Durchgangsinstrumente, insbesondere kein Elektrodeninstrument (20) und keine Optik (18), angeordnet sind.

9. Elektrochirurgisches System (38), wobei es ein Resektoskop (10) nach einem der Ansprüche 1 bis 8 und eine Spülflüssigkeitszufuhreinrichtung (40) umfasst, die mit dem Spülrohr (16) des Resektoskops (10) verbunden ist.

## Claims

1. A resectoscope (10) for endoscopic surgery, having a tubular shaft (12) which has an elongated sheath tube (14) and an irrigation tube (16) arranged in the sheath tube (14), which serves for the supply of irrigation fluid, as well as a rod-shaped lens (18) and an electrode instrument (20), wherein the lens (18) and the electrode instrument (20) are arranged between the outer wall (24) of the irrigation tube (16) and the inner wall (26) of the sheath tube (14), wherein the irrigation fluid supplied through the irrigation tube (16) tube into a body of a patient can be conveyed back out of the body through the sheath tube (14) and wherein the irrigation tube (16) has a cross section with a convexly curved portion (28) and a concavely curved portion (30), wherein the irrigation tube (16) has a nozzle (32) in its distal end region, by means of which a fluid flow flowing in the distal direction can be directed and accelerated.

2. The resectoscope (10) as claimed in claim 1, wherein the convexly curved portion (28) adjoins the inner wall (26) of the sheath tube (14).

3. The resectoscope (10) as claimed in any one of claims 1 to 2, wherein the irrigation tube (16) has a diffuser (34) in its distal end region.

4. The resectoscope (10) as claimed in any one of the preceding claims, wherein the electrode instrument (20) has one or more holding elements (36) for radial support.

5. The resectoscope (10) as claimed in claim 4, wherein the holding element or elements (36) adjoin the inner wall (26) of the sheath tube (14).

6. The resectoscope (10) as claimed in claim 4, wherein the holding element or elements (36) adjoin the outer wall (24) of the rod-shaped lens (18).

7. The resectoscope (10) as claimed in any one of the preceding claims, wherein the irrigation tube (16) has a cross section that is constant in size and shape over at least 60%, preferably at least 70%, of the length of the shaft (12).

8. The resectoscope (10) as claimed in any one of the preceding claims, wherein no passage instruments, in particular no electrode instrument (20) and no lens (18), are arranged within the irrigation tube (16).

9. An electrosurgical system (38), wherein it comprises a resectoscope (10) as claimed in any one of claims 1 to 8 and an irrigation fluid supply device (40) which is connected to the irrigation tube (16) of the resectoscope (10).

## Revendications

1. Résectoscope (10) pour la chirurgie endoscopique, avec une tige tubulaire (12), qui comprend une gaine (14) allongée et un tube de rinçage (16) disposé dans la gaine (14), qui sert à amener du liquide de rinçage, ainsi qu'une unité optique (18) en forme de tige et un instrument à électrode (20), l'unité optique (18) et l'instrument à électrode (20) étant disposés entre la paroi extérieure (24) du tube de rinçage (16) et la paroi intérieure (26) de la gaine (14), le liquide de rinçage amené dans le corps d'un patient à travers le tube de rinçage (16) pouvant être à nouveau évacué du corps à travers la gaine (14) et le tube de rinçage (16) comportant une section transversale avec une section bombée de manière convexe (28) et une section bombée de manière concave (30), le tube de rinçage (16) comportant, dans sa zone d'extrémité distale, une buse (32), au moyen de laquelle un écoulement de liquide s'écoulant dans la direction distale peut être dirigé et accéléré.

2. Résectoscope (10) selon la revendication 1, la section bombée de manière convexe (28) jouxtant la paroi intérieure (26) de la gaine (14).

3. Résectoscope (10) selon l'une des revendications 1 à 2, le tube de rinçage (16) comportant un diffuseur (34) dans sa zone d'extrémité distale.

4. Résectoscope (10) selon l'une des revendications précédentes, l'instrument à électrode (20) comportant un ou plusieurs éléments de maintien (36) pour l'appui radial.

5. Résectoscope (10) selon la revendication 4, le ou les éléments de maintien (36) jouxtant la paroi intérieure (26) de la gaine (14).

6. Résectoscope (10) selon la revendication 4, le ou les éléments de maintien (36) jouxtant la paroi extérieure (24) de l'unité optique (18) en forme de tige.

7. Résectoscope (10) selon l'une des revendications précédentes, le tube de rinçage (16) comportant une section transversale restant identique en taille et en forme sur au moins 60 %, de préférence au moins 70 %, de la longueur de la tige (12).

8. Résectoscope (10) selon l'une des revendications précédentes, aucun instrument de passage, en particulier aucun instrument à électrode (20) et aucune unité optique (18), n'étant disposé à l'intérieur du tube de rinçage (16).

9. Système électrochirurgical (38), ledit système comprenant un résectoscope (10) selon l'une des revendications 1 à 8 et un dispositif d'amenée de liquide de rinçage (40), qui est relié au tube de rinçage (16) du résectoscope (10).
